# EUROPEAN PATENT APPLICATION

(11) **EP 0 554 595 A1**
(43) Date of publication of application: **11.08.1993**
(21) Application number: 92300995.5
(22) Date of filing: 06.02.1992
(51) Int. Cl.: C09D 5/08, C09D 5/44, C07D 277/70

(54) **Manufacture of surface coatings**

(71) Applicant: CIBA-GEIGY AG, CH-4002 Basel (CH)
(72) Inventor: Braig, Adalbert, W-7858 Weil-Friedlingen (DE); Phillips, Emyr, Wakefield, Yorkshire (GB)
(74) Representative: Sparrow, Kenneth D.

(57) **Abstract**

The present invention provides a method for the manufacture of a corrosion-inhibiting surface coating comprising reacting a film-forming binder component of the surface coating medium with a compound having the formula IA or IB: 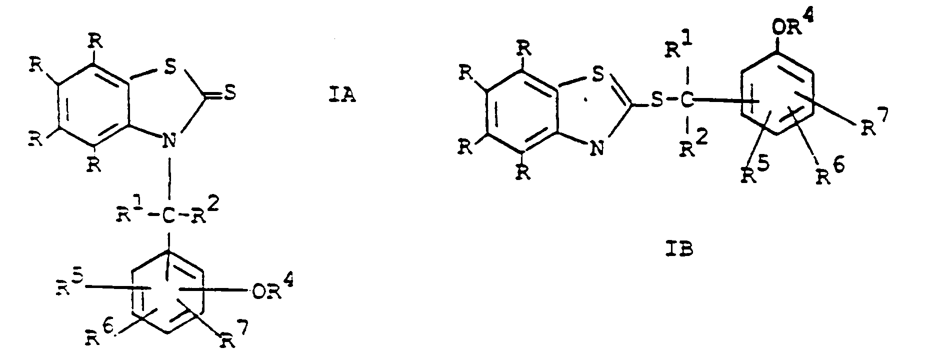
wherein each R, independently, is hydrogen, C₁-C₁₂ alkyl, C₃-C₂₀ alkenyl, C₁-C₄ halogenoalkyl, C₁-C₁₂ alkoxy, C₁-C₁₂ alkylthio, phenylthio, benzylthio, C₁-C₁₂ alkylsulphonyl, phenyl, C₇-C₁₅ alkylphenyl, C₇-C₁₀ phenylalkyl, C₅-C₈ cycloalkyl, halogen, nitro, cyano, carboxyl, COO(C₁-C₄ alkyl), hydroxy, amino, NHR³, N(R³)₂, CONH₂, CONHR³, CON(R³)₂, C₁-C₁₂ alkyl interrupted by an O-, N- or S-atom, NCO, C₁-C₄ alkylene-NCO, epoxy, C₁-C₄ alkyleneepoxy, CHO or COR³;
R¹ is hydrogen, C₁-C₁₂ alkyl, phenyl optionally substituted by halogen, C₁-C₄ alkyl, C₁-C₄ alkoxy or nitro, or R¹ is pyridyl, thienyl or furyl;
R² is hydrogen or C₁-C₄ alkyl;
R³ is C₁-C₁₂ alkyl, C₃-C₁₂ alkyl interrupted by one or more O-atoms, C₅-C₈ cycloalkyl, benzyl, phenyl optionally substituted by halogen, C₁-C₄ alkyl, C₁-C₄ alkoxy or nitro, or two groups R³, together with an N-atom to which they are attached, can form a pyrrolidino, piperidino or morpholino residue;
R⁴ is hydrogen, (CH₂)m CO₂H, (CH₂)mCO₂R⁸, 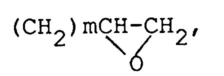
(CH₂)mNCO, (CH₂)mOH, (CH₂)mOSi(CH₃)₃, Si(CH₃)₃, SO₂R⁸, CONHR³, CON(R³)₂, C(=S)NHR³ or C(=S)N(R³)₂;
R⁵ and R⁶, independently, are hydrogen, halogen, C₁-C₄ alkoxy, cyano, nitro, C₁-C₂₀ alkyl, (CH₂)nCO₂R⁸, (CH₂)nCONHR³, (CH₂)nCON(R³)₂, C₃-C₂₀ alkenyl, C₇-C₁₀ phenylalkyl phenyl, C₅-C₈ cycloalkyl or a group of formula IIA or IIB: 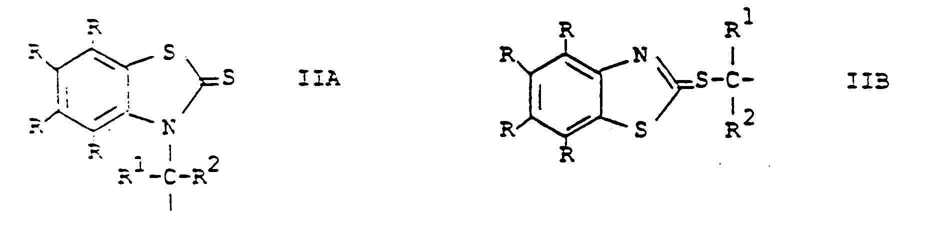
wherein R, R¹ and R² have their previous significance;
R⁷ is hydrogen, C₁-C₂₀ alkyl, C₁-C₂₀ alkyl interrupted by an O-, N- or S-atom, C₃-C₂₀ alkenyl, 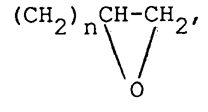
(CH₂)ₙ OH, hydroxy (CH₂)ₙ NCO, (CH₂)ₙCO₂H, (CH₂)ₙCO₂R⁸, (CH₂)ₙSi(OCH₃)₃ or CHO, or R⁷ is a residue of formula: 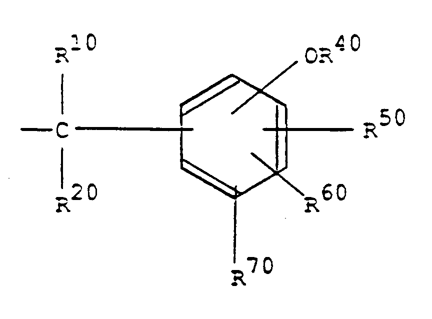
in which R¹⁰ has one of meanings of R¹ but does not necessarily have the identical meaning, R²⁰ has one of the meanings of R² but does not necessarily have the identical meaning, R⁴⁰ has one of the meanings of R⁴ but not necessarily the same meaning, R⁵⁰has one of the meanings, but not necessarily the same meaning as R⁵, R⁶⁰ has one of the meanings, but not necessarily the same meaning as R⁶, and R⁷⁰ has one of the meanings but not necessarily the same meaning as R⁷, or R⁵ and R⁷ , or R⁶ and R⁷ can form, fused to the phenol or phenoxy ring, a carbocyclic or heterocyclic ring, the heteroatoms being O, N or S, and the ring being optionally substituted with a C₁-C₄alkyl, C₁-C₄ alkoxy or halogen substituent, provided that, when R⁴ is hydrogen, R⁷ is one of the defined meanings other than hydrogen or C₁-C₂₀ alkyl;
R⁸ is C₁-C₂₀ alkyl optionally substituted by halogen or hydroxy, or is C₃-C₂₀ alkyl which is interrupted by one or more O-atoms and is substituted by one or more of hydroxy, phenyl, C₇-C₁₅ alkylphenyl or C₇- C₁₀ phenylalkyl; m is an integer from 1 to 20; and n is O or an integer from 1 to 20.

## Description

The present invention relates to a method for the manufacture of a corrosion-inhibiting surface coating; and to new corrosion inhibiting reactants for use in the new method.

Protection against corrosion is one of the most important functions of organic coating compositions for metal substrates. Many suggestions for improving the protection of coatings against corrosion are to be found in the literature, for example in H. Kittel, Lehrbuch der Lacke und Beschichtungen ("Textbook of Paints and Coatings"), volume V, Stuttgart 1977, 46-103.

On the one hand, the barrier function of the coating composition can be improved, in order to keep corrosive agents, such as oxygen, water and ions, away from the metal surface. On the other hand, it is possible to employ corrosion-inhibiting pigments which intervene chemically or electrochemically in the corrosion process, for example by the formation of insoluble deposits with corrosion products or by passivation (polarisation) of the metal surface. Metal chromates and lead compounds rank amongst the most effective corrosion-inhibiting pigments. Much use has been made of metal chromates, particularly because they inhibit both anodic and cathodic corrosion. Nowadays there are certain objections to the use of chromates owing to their potential carcinogenic action. Similarly, there are objections to the use of lead compounds owing to their chronic toxicity.

These objections to the use of toxic inorganic corrosion inhibitors have been overcome by the use of certain organic compounds containing a phenol moiety as described in Cases EP-A-259254 and EP-A-259255.

Conventionally, corrosion inhibitors have been added to a surface coating after the synthesis of the binder component of the surface coating.

In a further development in this art, we have now provided a new method for the manufacture of a corrosion-inhibiting surface coating medium. The new method guarantees that the corrosion inhibitor is placed in contact with the metal surface to be protected as part of the surface coating medium, so that binding of the corrosion inhibitor to the metal surface can be enhanced. In addition, secondary problems e.g. interactions or incompatibility with resin systems, in particular with water-dispersible resin systems, a pH change of such systems, and significant changes of the bath conductivity of electro-depositable systems, leading to different application conditions, are avoided.

Accordingly, the present invention provides a method for the manufacture of a corrosion-inhibiting surface coating comprising reacting a film-forming binder component of the surface coating medium with a compound having the formula IA or IB: 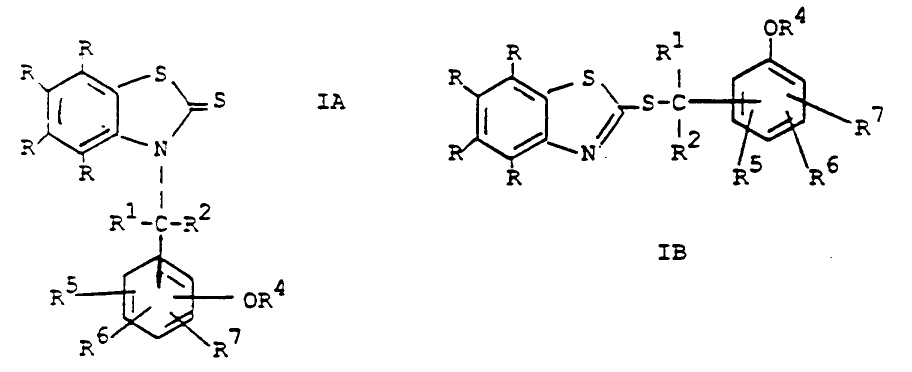
wherein each R, independently, is hydrogen, C₁-C₁₂ alkyl, C₃-C₂₀ alkenyl, C₁-C₄ halogenoalkyl, C₁-C₁₂ alkoxy, C₁-C₁₂₋ alkylthio, phenylthio, benzylthio, C₁-C₁₂ alkylsulphonyl, phenyl, C₇-C₁₅ alkylphenyl, C₇-C₁₀ phenylalkyl, C₅-C₈-cycloalkyl, halogen, nitro, cyano, carboxyl, COO(C₁-C₄ alkyl), hydroxy, amino, NHR³, N(R³)₂, CONH₂, CONHR³, CON(R³)₂, C₁-C₁₂ alkyl interrupted by an O-, N- or S-atom, NCO, C₁-C₄-alkylene-NCO, epoxy, C₁-C₄ alkylene-epoxy, CHO or COR³;
R¹ is hydrogen, C₁-C₁₂ alkyl, phenyl optionally substituted by halogen, C₁-C₄ alkyl, C₁-C₄ alkoxy or nitro, or R¹ is pyridyl, thienyl or furyl;
R² is hydrogen or C₁-C₄ alkyl;
R³ is C₁-C₁₂ alkyl, C₃-C₁₂ alkyl interrupted by one or more O-atoms, C₅-C₈ cycloalkyl, benzyl, phenyl optionally substituted by halogen, C₁-C₄ alkyl, C₁-C₄ alkoxy or nitro, or two groups R³, together with an N-atom to which they are attached, can form a pyrrolidino, piperidino or morpholino residue;
R⁴ is hydrogen, (CH₂)m CO₂H, (CH₂)mCO₂R⁸, 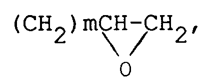
(CH₂)mNCO, (CH₂)mOH, (CH₂)mOSi(CH₃)₃, Si(CH₃)₃, SO₂R⁸, CONHR³, CON(R³)₂, C(=S)NHR³ or C(=S)N(R³)₂;
R⁵ and R⁶, independently, are hydrogen, halogen, C₁-C₄ alkoxy, cyano, nitro, C₁-C₂₀ alkyl, (CH₂)nCO₂R⁸, (CH₂)nCONHR³, (CH₂)nCON(R³)₂, C₃-C₂₀ alkenyl, C₇-C₁₀ phenylalkyl, phenyl, C₅-C₈ cycloalkyl or a group of formula IIA or IIB: 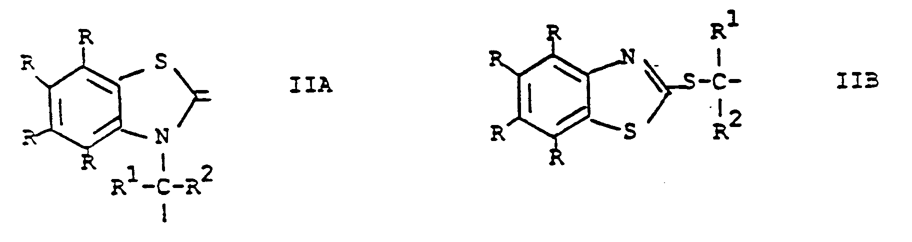
wherein R, R¹ and R² have their previous significance;
R⁷ is hydrogen C₁-C₂₀ alkyl, C₁-C₂₀ alkyl interrupted by an O-, N- or S-atom, C₃-C₂₀ alkenyl, 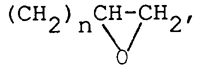
(CH₂)ₙ OH hydroxy, (CH₂)ₙNCO, (CH₂)ₙCO₂H, (CH₂)ₙCO₂R⁸, (CH₂)ₙSi(OCH₃)₃ or CHO, or R⁷ is a residue of formula: 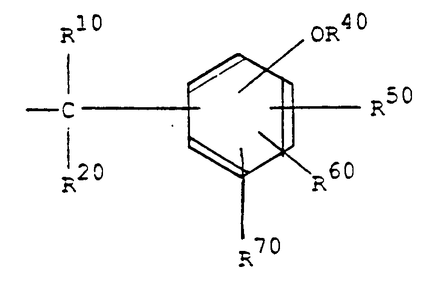
in which R¹⁰ has one of meanings as R¹ but does not necessarily have the identical meaning, R²⁰ has one of the meanings of R² but does not necessarily have the identical meaning, R⁴⁰ has one of the meanings of R⁴ but not necessarily the same meaning, R⁵⁰ has one of the meanings, but not necessarily the same meaning as R⁵, R⁶⁰ has one of the meanings but not necessarily the same meaning as R⁶, and R⁷⁰ has one of the meanings but not necessarily the same meaning as R⁷ or R⁵ and R⁷, or R⁶ and R⁷ can form, fused to the phenol or phenoxy ring, a carbocyclic or heterocyclic ring, the heteroatoms being O, N or S, and the ring being optionally substituted with a C₁-C₄ alkyl, C₁-C₄ alkoxy or halogen substituent, provided that, when R⁴ is hydrogen, R⁷ is one of the defined meanings other than hydrogen or C₁-C₂₀ alkyl; R⁸ is C₁-C₂₀ alkyl optionally substituted by halogen or hydroxy, or is C₃-C₂₀ alkyl which is interrupted by one or more O-atoms and is substituted by one or more of hydroxy, phenyl, C₇-C₁₅ alkylphenyl or C₇-C₁₀ phenylalkyl; m is an integer from 1 to 20; and n is O or an integer from 1 to 20.

The film-forming binder reactant may be any film-former suitable for solvent-based, but in particular aqueous-based coating compositions. Examples of such film-forming binders are epoxide resins, polyurethane resins, aminoplast resins, or mixtures of such resins; or a basic aqueous dispersion or solution of an acidic resin.

Of particular interest are film-forming reactants which are binders for aqueous-based coating compositions e.g. alkyld resins; acrylic resins; two-pack epoxy resins; polyester resins which are usually saturated; water-dilutable phenolic resins or dispersions thereof; water-dilutable urea resins; and vinyl/acrylic copolymer resins.

In a preferred embodiment, a compound of formula IA or IB, is incorporated into the polymer skeleton of an epoxide resin by either a) incorporating the compound of formula IA or IB, into the epoxide resin by partial replacement of the bisphenol A part of the resin or by partial replacement of the polyol component; or b) incorporation into the blocked polyisocyanate (cross-linking component), as a blocking agent, with the object of a controlled release during the stoving operation.

The choice of particular compound of formula IA or IB, reactant, will depend on the nature of reactive groups present in the film-forming binder reactant.

To take one group of film-forming binder reactants, by way of example, epoxide resins are formed by the reaction of compounds containing a 1,2-ethylene oxide group with compounds containing active hydrogen atoms, e.g. amines, acids, phenols, thiols and alcohols.

In order to chemically incorporate a compound of formula IA or IB into an epoxide resin film-former, therefore, a suitable compound of formula IA or IB reactant would be one which a) contains one or more of an amino-, acidic-, phenolic-, thiolic- or alcoholic group, in order to react with an epoxide precursor, and/or b) contains a 1,2-ethylene oxide group in order to react with an amine, acid, phenol, thiol or alcohol precursor.

By means of the new method of the present invention, therefore, a surface coating is obtained in which the corrosion inhibitor moiety is actually built into the structure of the film-forming binder component of the surface coatings so produced.

More specifically, the alkyd resins may be water-dilutable alkyds such as air-drying or bake systems which may also be used in combination with water-dilutable melamine resins; or alkyd emulsions either oxidatively- or air-drying or bake systems, optionally used in combination with water-borne acrylics or copolymers thereof, vinyl acetates etc. Acrylic resin reactants may be straight acrylics; acrylic acid ester copolymers; combinations or copolymers with vinyl resins e.g. vinyl acetate, or with styrene. These systems may be air- drying or bake systems.

Water-dilutable epoxide resin reactants, in combination with suitable polyamine curing agents have good mechanical and chemical stability. By the polyaddition of epoxide resin with amine, thermosets are obtained having very high film hardness.

The addition of organic solvents is not necessary when liquid epoxy-based resins are used for aqueous systems. When using epoxide-solid resin dispersions, a minor amount of solvent is necessary for improved film formation.

Preferred epoxide resin reactants are those based on aromatic polyols, in particular bisphenols. The epoxide resins are used in conjunction with a curing agent. The latter can be, in particular, an amino or hydroxy compound or an acid or an acid anhydride or a Lewis acid. Examples of these are polyamines, polyaminoamides, polysulfide polymers, polyphenols, boron fluoride and complexes thereof, polycarboxylic acids, 1,2-dicarboxylic acid anhydrides or pyromellitic dianhydride.

In formula IA or IB, R, R¹, R², R³, R⁵, R⁶, R⁷ or R⁸ can, as alkyl, be unbranched or branched alkyl. If this is C₁-C₄ alkyl, it can be, for example, methyl, ethyl, n-propyl, isopropyl, n-butyl, sec.-butyl, isobutyl or tert.-butyl. R, R¹, R³, R⁵, R⁶, R⁷ and R⁸ can also be C₅-C₁₂ alkyl, for example pentyl, hexyl, n-octyl, 2-ethylhexyl, 1,l,3,3-tetramethylbutyl, 1,1,3,3,5,5-hexamethylhexyl, n-decyl, isodecyl or n-dodecyl. R⁵, R⁶, R⁷ and R⁸ can also be C₁₃-C₂₀ alkyl, for example, tridecyl, tetradecyl, pentadecyl, hexadecyl, heptadecyl, octadecyl, nonadecyl or eicosyl.

As C₃-C₂₀ alkenyl, R, R⁵, R⁶ and R⁷ can, for example, by allyl, methallyl, 2-butenyl, 2-hexenyl, undecenyl, pentadecenyl, octadecenyl(oleyl) or decenyl.

As halogenoalkyl, R can, for example, by chloromethyl, trichloromethyl, bromomethyl, 2-chloroethyl, 2,2,2-trichloromethyl, trifluoromethyl or 2,3-dichloropropyl.

As alkoxy, alkylthio or alkylsulfonyl, R can, for example, be methoxy, ethoxy, isopropoxy, butoxy, hexyloxy, octyloxy, dodecyloxy, methylthio, tert.butylthio, dodecylthio, methylsulfonyl, ethylsulfonyl, hexylsulfonyl or dodecylsulfonyl. As alkylphenyl, R can, for example, by tolyl, xylyl, 4-ethylphenyl, 4-tert.butylphenyl, 4-octylphenyl or 4-nonylphenyl.

As phenylalkyl, R, R¹, R³, R⁵ and R⁶ can, for example, be benzyl, 1-phenylethyl, 2-phenylethyl, α,α-dimethylbenzyl or 2-phenylpropyl.

As cycloalkyl, R, R³, R⁵ and R⁶ can, for example, be cyclopentyl, cyclohexyl, cycloheptyl, methylcyclohexyl or cyclooctyl.

As phenyl which is substituted by halogen, C₁-C₄ alkyl, C₁-C₄ alkoxy or nitro, R¹, R³, can, for example, be 4-chlorophenyl, 3-bromophenyl, 2-fluorophenyl, p-tolyl, 3,5-dimethylphenyl, 4-isopropylphenyl, 4-methoxyphenyl, 3-ethoxyphenyl, 4-nitrophenyl or 4-nitro-2-methylphenyl.

As alkyl which is interrupted by O, R³ and R⁸ can, for example be 2-methoxyethyl, 2-butoxyethyl, 3,6-dioxaheptyl or 3,6-dioxadecyl. R⁷ may also be a polyethylene glycol residue having up to 20 C-atoms and up to 10 O-atoms.

When R⁵ and R⁷ together or R⁶ and R⁷ together form a ring fused to the phenolic moiety, the ring so formed is preferably a pyridine ring, a benzene ring or benzofuran ring, so producing a naphthol moiety, a hydroxyquinoline moiety or a hydroxydibenzofuran moiety.

Compounds of the formula IA or IB in which one R is hydrogen, C₁-C₄ alkyl, C₁-C₄ alkoxy, trifluoromethyl, halogen or nitro and the other three R's are hydrogen, are preferred.

Compounds of the formula IA or IB, in which R¹ is hydrogen, C₁-C₈ alkyl, phenyl or furyl and R² is hydrogen, especially a compound of the formula I in which R¹ and R² are hydrogen, are also preferred.

Compounds of the formula IA or IB are also preferred in which R⁴ has its previous significance, R⁵ and R⁶ independently are hydrogen, C₁-C₈ alkyl, allyl, C₇-C₁₀ phenylalkyl, C₁-C₄ alkoxy, halogen, phenyl, cyclohexyl or a group -CH₂CH₂COOR⁸, in which R⁸ is C₁-C₁₈ alkyl or C₃-C₁₀ alkyl which is interrupted by one or more oxygen atoms, and R⁷ is hydrogen, C₁-C₁₈ alkyl or C₃-C₂₀ alkenyl, provided that when R⁴ is hydrogen, then R⁷ is C₃-C₁₈ alkenyl,

Examples of individual compounds of the formula IA or IB in which R¹=R²=H are those containing the substituents indicated below:

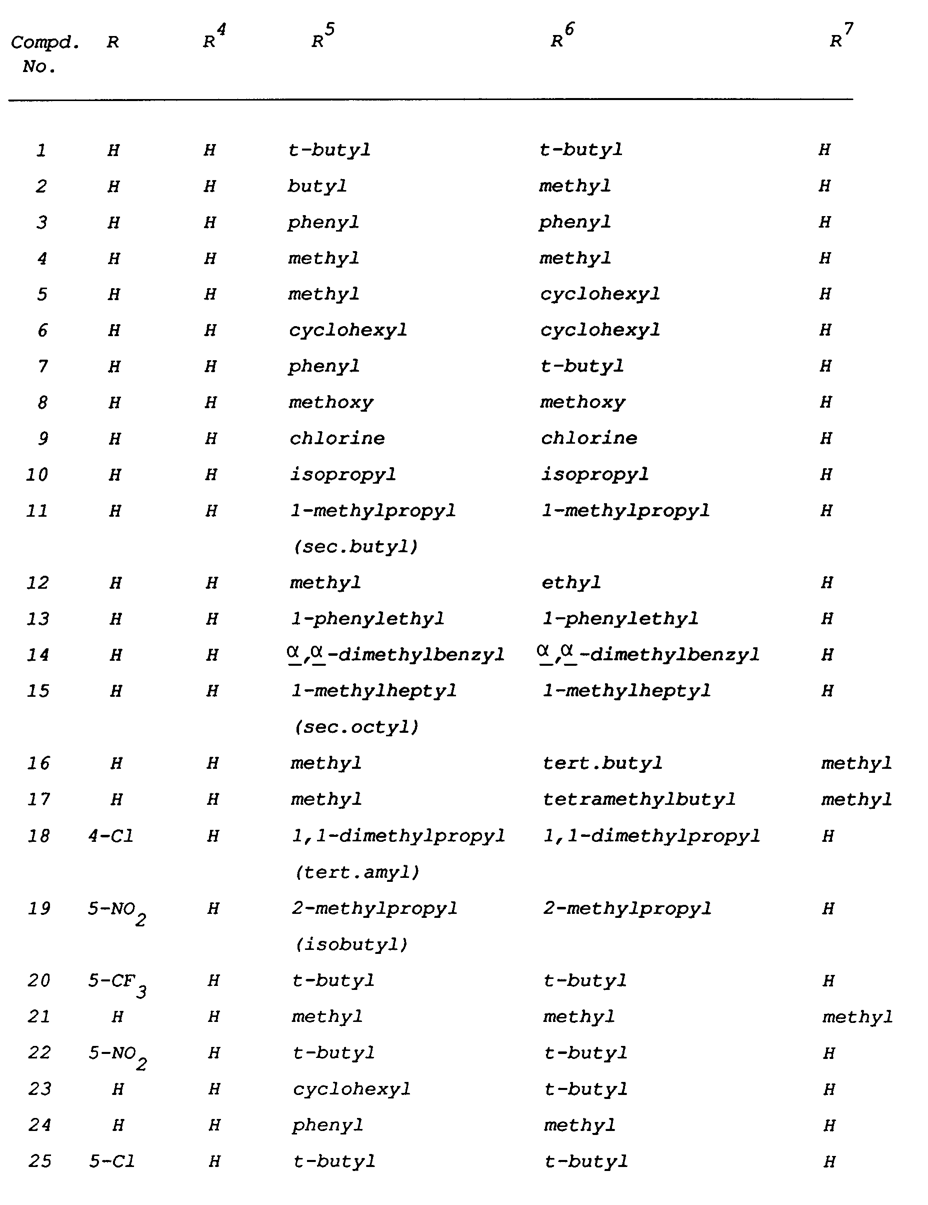

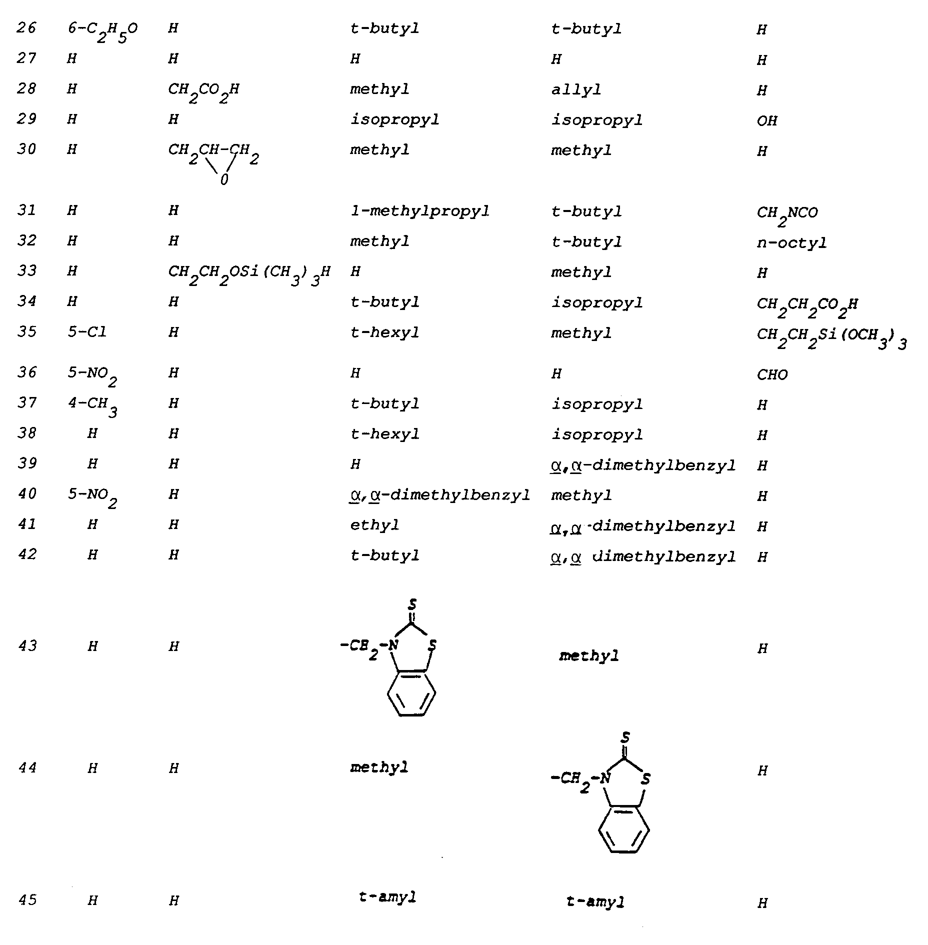

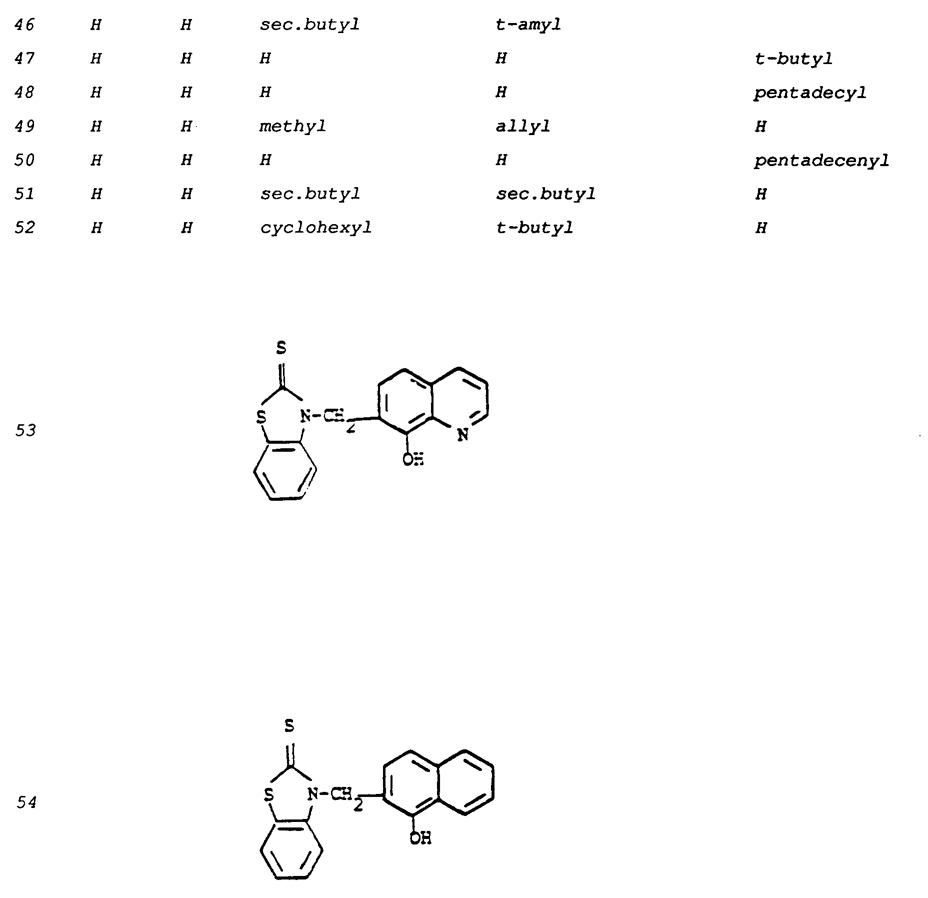

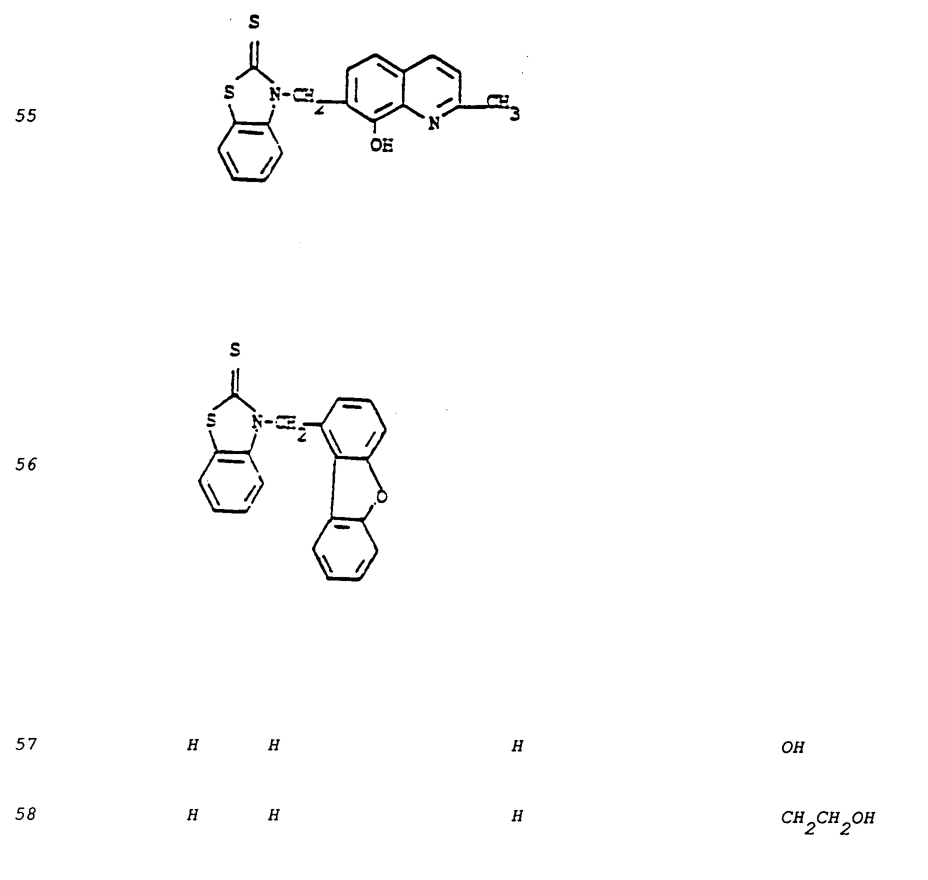

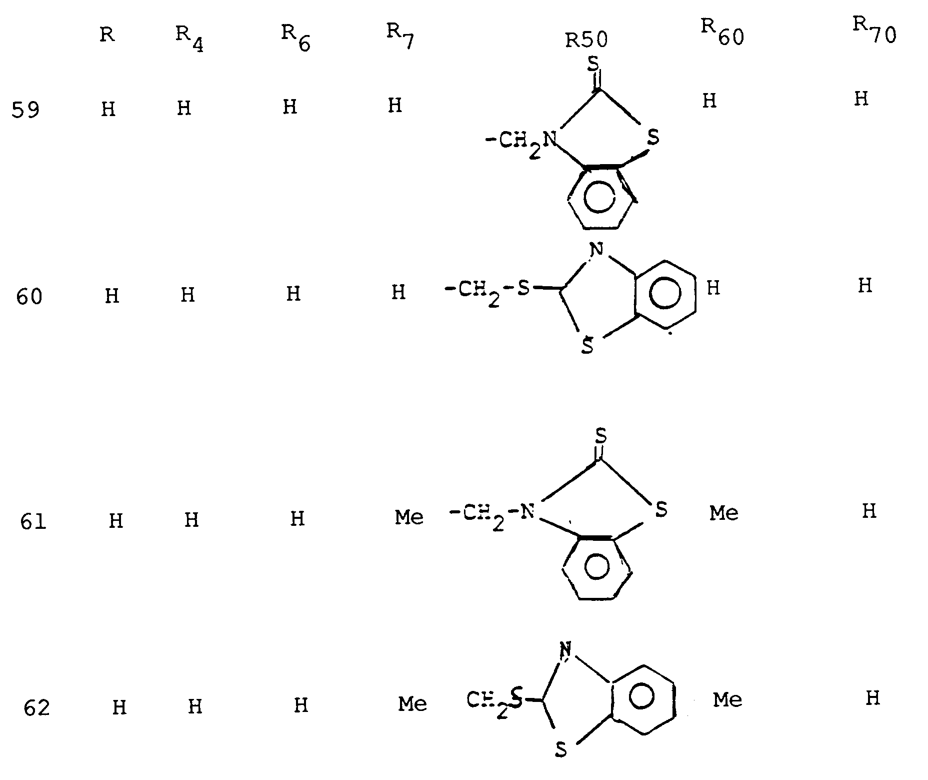

The preparation of the compounds of formula IA can be effected by heating the corresponding S-substituted isomers of the formula IB: 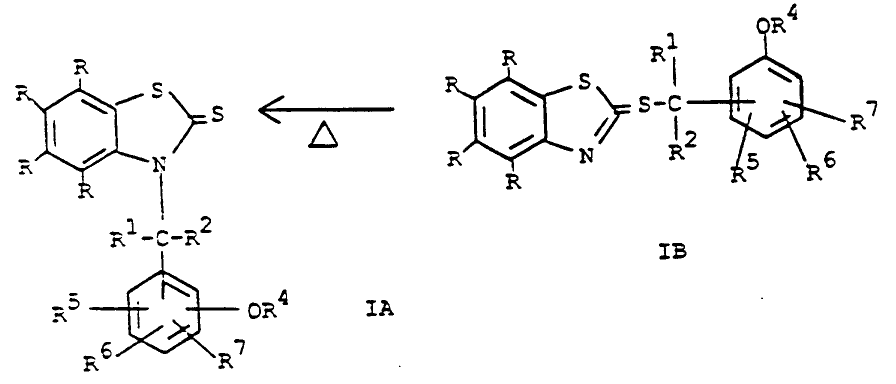
Heating can be carried out with or without a solvent. Examples of suitable solvents are aromatic hydrocarbons, such as toluene or xylene; halogenated hydrocarbons, such as tetrachloroethylene or chlorobenzene; alkanols, such as isopropanol or n-butanol; or esters, ketones, dimethylformamide or dimethyl sulfoxide. Polar solvents, for example dimethylformamide, accelerate the reaction. The rearrangement can also be accelerated by adding basic catalysts. Examples of the latter are, in particular, aliphatic, cycloaliphatic or heterocyclic amines. If the phenolic OR⁴ group is in the para position relative to the radical C (R¹)(R²), the rearrangement proceeds more rapidly than if the group is in the ortho-position. The temperature required for the rearrangement therefore depends on the position of the OH group and on the solvent and catalyst used. It is preferably carried out at 70-250°C, in particular at 100-200°C.

Compounds of formula IB may be produced by reacting the corresponding 2-mercaptobenzothiazoles VI with a carbonyl compound VII and a phenol VIII with acid catalysis, e.g. in accordance with the equation. 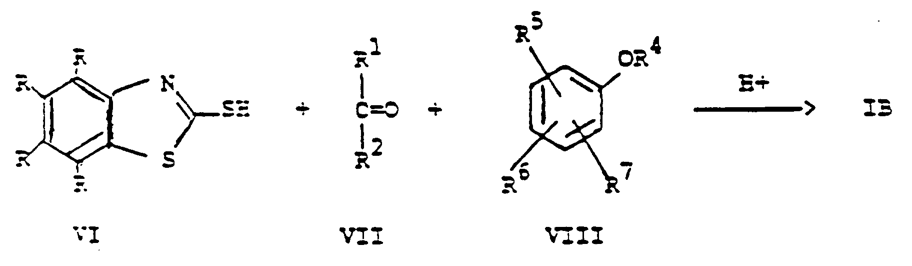
as described, for example, in US Patent 3281473.
Alternatively, compounds of formula IB can also be prepared from VI by reacting the latter with the corresponding benzyl alcohol IX: 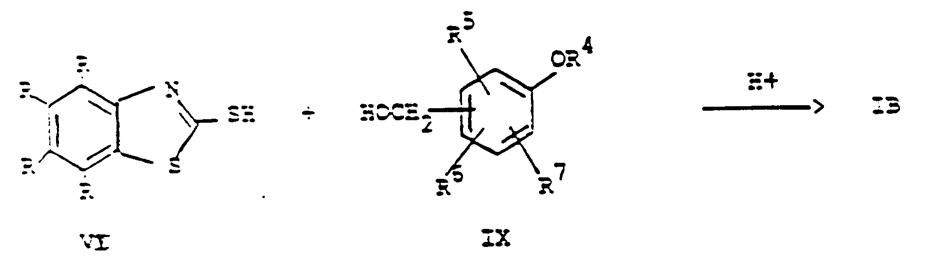
as described, for example, in US Patent 3215641.

A second possible means of preparing compounds of the formula IA is the reaction of 2-mercaptobenzothiazoles of the formula VI with an N-disubstituted aminomethylphenol of the formula X: 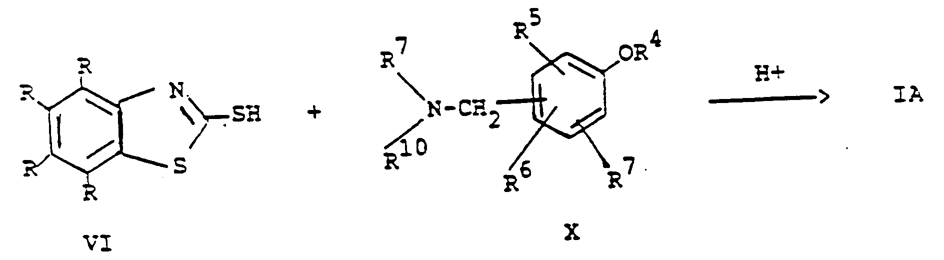
In these formulae, R⁹ and R¹⁰ independently of one another are C₁-C₁₂ alkyl, C₅-C₈ cycloalkyl, benzyl or phenyl. This reaction is described in USSR Patent 1164233. It is preferably carried out in a polar organic solvent. Examples of these are lower alkanols (C₁-C₄), dimethylformamide or dimethyl sulfoxide. The reaction is carried out by heating at 50-200°C, preferably 70-150°C.

A third possible method of preparation is the reaction of a 2-mercaptobenzothiazole of the formula VI with a carbonyl compound of the formula VII and a phenol of the formula VIII with base catalysis: 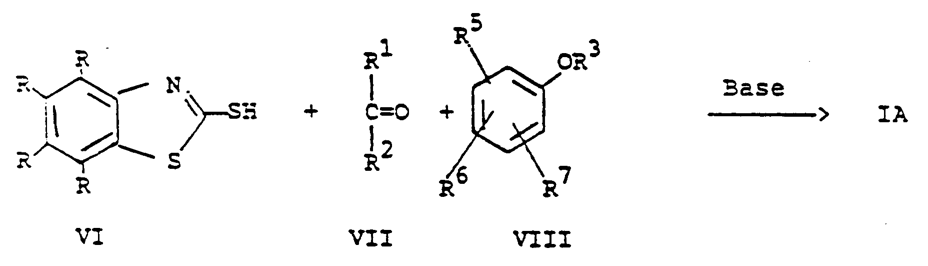
Whereas, as previously described, the S-substituted isomers of the formula IB are formed with acid catalysis, the N-substituted isomers of the formula I are formed in the same reaction with base catalysis.

The reaction is preferably carried out in a polar solvent by heating at 50-150°C, preferably 70-120°C.

Suitable basic catalysts are any known organic or inorganic strong bases. It is preferable to use primary, secondary or tertiary amines, for example isopropylamine, butylamine, cyclohexylamine, dibutylamine, dihexylamine, di(isopropyl)-amine, triethylamine, tributylamine, piperidine, morpholine, pyrrolidine or quinoline. The reaction is particularly suitable if formaldehyde is used as the carbonyl compound, with the formation of products of the formula IA in which R¹ = R² = H. The formaldehyde can be used, for example as an aqueous solution (formalin) or in the form of paraformaldehyde, or a reagent which forms formaldehyde under the reaction conditions, for example hexamethylenetetramine, is used.

This reaction is also suitable for the preparation of compounds of the formula IA in which R⁵ or R⁶ is a group of formula IIA. In this case use is made of a phenol of formula VIII in which R⁵ or R⁶ is hydrogen and it is reacted with at least two equivalents of mercaptobenzothiazole of the formula VI and at least two equivalents of the carbonyl compound of the formula VII: 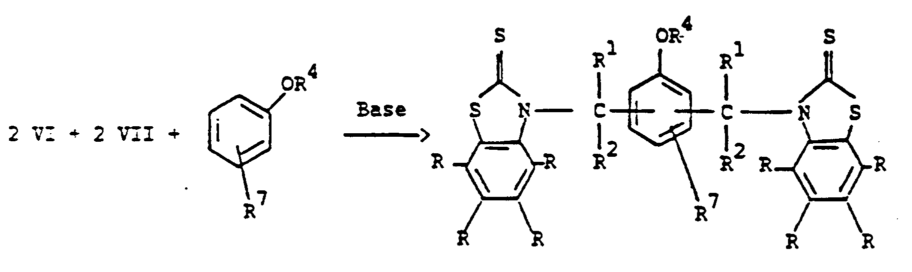
Surface coatings produced by the method according to the present invention may be formulated to include further components. Examples of further components, include for example pigments, dyes, extenders and other additives such as are customary for coating compositions. The pigments can be organic, inorganic or metallic pigments, for example titanium dioxide, iron oxide, aluminium bronze, phthalocyanine blue etc. It is also possible to use concomitantly anti-corrosion pigments, for example pigments containing phosphates or borates, metal pigments and metal oxide pigments (see Farbe und Lack 88 (1982)), 183) or the pigments described in European Patent A 54,267. Examples of extenders which can be used concomitantly are talc, chalk, alumina, baryte, mica or silica. Examples or further additives are flow control auxiliaries, dispersing agents, thixotropic agents, adhesion promoters, antioxidants, light stabilisers or curing catalysts.

Particular importance attaches to the addition of basic extenders or pigments. In certain binder systems, for example in acrylic and alkyd resins, these produce a synergistic effect on the inhibition of corrosion. Examples or such basic extenders or pigments are calcium carbonate, magnesium carbonate, zinc oxide, zinc carbonate, zinc phosphate, magnesium oxide, aluminium oxide, aluminium phosphate or mixtures thereof. Examples of pigments are those based on aminoanthraquinone.

The coating composition can also contain further organic, metal-organic or inorganic corrosion inhibitors, for example salts of nitroisophthalic acid, tannin, phosphoric esters, technical amines, substituted benztriazoles or substituted phenols, such as are described in German Offenlegungsschrift 3,146,265.

Finally, any further corrosion inhibitor can also be applied to a neutral carrier. Suitable carriers are, in particular, pulverulent extenders or pigments. This technique is described in greater detail in German Offenlegungsschrift 3,122,907.

The coating compositions produced according to the invention are preferably used as a primer on metallic substrates, in particular on iron, steel, copper, aluminium, aluminium alloys or zinc. Here they can function as so-called conversion coatings, in that chemical reactions take place at the interface between the metal and the coating. The application of the coatings can be effected by the customary methods, such as spraying, brushing, roller-coating, dipping or electrodeposition, in particular cathodic deposition. Depending on whether the film-former is a resin which dries physically or can be cured by heat or radiation, the curing of the coatings is carried out at room temperature, by stoving or by irradiation.

Any further corrosion inhibitors can be added to the coating composition during the preparation of the latter, for example during the distribution of the pigment by grinding. The inhibitor is used in an amount of 0.01 - 20% by weight, preferably 0.5 - 5% by weight, based on the solids content of the coating composition.

The production of surface coatings by electrodeposition viz. the deposition of a film-forming material under the influence of an applied electrical potential is well established commercially. Various coating materials have been developed for this method of application. Using this method, it is difficult to get the desired overall corrosion protection without using certain toxic anticorrosive pigments viz. lead pigments. More specifically, it is difficult to achieve sufficient overall corrosion protection/properties by the (optionally pigmented) resin technology alone.

We have now found that the coating compositions produced according to the method of the present invention impart excellent corrosive-inhibiting properties to both cathodic and anodic electrocoats.

As film-forming reactant of the electrodepositable cathodic aqueous coating compositions produced according to the method of the present invention, there may be used e.g. an epoxy resin optionally crosslinked with a capped or blocked organic polyisocyanate; acrylic resins optionally and preferably crosslinked with a capped or blocked isocyanate; acrylic or other unsaturated resins crosslinked via double bonds; adducts of epoxy resins with amines, polycarboxylic acids or their anhydrides or aminocarboxylic, mercaptocarboxylic or aminosulphonic acids; polyurethanes; polyesters; and reaction products of phenolic hydroxyl group-containing resins with an aldehyde and an amine or amino- or mercapto- carboxylic or aminosulphonic acid; as well as mixtures of these resins.

Preferred adducts of an epoxide resin with an amine are adducts of a polyglycidyl ether, which may be of a polyhydric phenol or a polyhydric alcohol, with a monoamine. Suitable polyglycidyl ethers include those of dihydric alcohols such as butane-1,4-diol, neopentyl glycol, hexamethylene glycol, oxyalkylene glycols and polyoxyalkylene glycols, and trihydric alcohols such as glycerol, 1,1,1-trimethylolpropane and adducts of these alcohols with ethylene oxide or propylene oxide. It will be understood by those skilled in the art that these polyglycidyl ethers of polyhydric alcohols are usually advanced, i.e. converted into longer chain higher molecular weight polyglycidyl ethers, for example by reaction with a dihydric alcohols or phenol, so that the resulting polyglycidyl ethers given adducts with suitable electrodepositable film-forming properties on reaction with the secondary monoamine. Preferred polyglycidyl ethers are those of polyhydric phenols, including bisphenols such as bisphenol F, bisphenol A and tetrabromobisphenol A and phenolic novolak resins such as phenol-formaldehyde or cresol-formaldehyde novolak resins. These polyglycidyl ethers of phenols may have been advanced, for example by reaction with dihydric alcohols or phenols such as those hereinbefore described. Particularly preferred polyglycidyl ethers are polyglycidyl ethers of bisphenol A advanced by reaction with bisphenol A.

Monoamines suitable for adduct formation with the polyglycidyl ethers include primary, secondary or tertiary amines. Secondary amines are preferred e.g. dialkylamines such as diethylamine, di-n-propylamine, di-isopropylamine, di-n-butylamine, di-n-octylamine and di-n-dodecylamine or nitrogen heterocycles such as piperidine or morpholine.

Preferred secondary monoamines are secondary alkanolamines such as diethanolamine, N-methylethanolamine, N-butylethanolamine, diisopropanolamine, N-methylisopropanolamine or di-n-butanolamine. A particularly preferred secondary alkanolamine is diethanolamine.

Thus preferred adducts of polyglycidyl ether with a secondary monoamine are adducts of a polyglycidyl ether of a polyhydric phenol, which may have been advanced, with a secondary alkanolamine, while particularly preferred such adducts are those of a polyglycidyl ether of bisphenol A, advanced by reaction with bisphenol A, with diethanolamine.

Electrodeposition of the organic resin may be carried out using conventional procedures.

Any further corrosion inhibitor component b) may be added to the electrodepositable coating system during the preparation of the latter, for example, during the distribution of the pigment by grinding e.g. by the methods disclosed in EP 107089. Alternatively, any further corrosion inhibitors can be incorporated into the non-emulsified resins and also into the grind resin. The further corrosion inhibitors are preferably used in an amount of 0.01 to 20 % by weight, preferably 0.05 to 5 % by weight, based on the solids content of the electrodepositable coating composition.

Electrodeposition for only a few minutes, usually one minute, at a voltage of up to 500 volts is sufficient in most cases. Usually, voltage programs, viz. stepwise increase of the voltage, are used.

The coating compositions of the present invention may be applied to any electrically conductive substrate especially metals such as iron; steel; e.g. cold-rolled steel, optionally treated with zinc phosphate or galvanized; copper; zinc; and aluminium; more especially zinc or aluminium alloys.

After electrodeposition of the organic resin film, the substrate is rinsed with de-mineralized water, air-blasted and baked at elevated temperatures e.g. up to 500 F

The following Example further illustrates the present invention. Example 1 relates to the preparation of a control electrodip lacquer.

### Example 1 Production of Conventional Electrodip Lacquer

An electrodip lacquer is prepared according to the procedure described in Example II of U.S. Patent Specification number 4148772.
A) 970 Parts by weight of an epoxy resin having an epoxy equivalent weight of 485, and 265 parts by weight of a polycaprolactone diol are charged into a reactor. The charge is heated to 100^{o}C under a nitrogen blanket and 0.46 part of benzyl dimethylamine is added. The reaction mixture is heated further to 130^{o}C, where it is held for about one and a half hours. The batch is cooled to 110^{o}C and 110 parts by weight of methyl isobutyl ketone are introduced into the reaction vessel, followed by 39.8 parts by weight of a 73 percent non-volatile solution of methyl isobutyl diketimine of diethylene triamine in methyl isobutyl ketone solvent, followed by an additional 100 parts by weight of methyl isobutyl ketone. Cooling is continued until the batch reaches 70^{o}C, at which point 55.1 parts by weight of diethyamine are introduced and the batch is reheated to 120^{o}C where it is held for 3 hours and discharged.
B) To prepare the chain extended electrodepositable thermosetting cationic urethane modified composition, 576 parts of the above-described polycaprolactone diol chain extended polyether is blended with 310 parts by weight of a polyurethane crosslinker and 13.2 parts of dibutyltin dilaurate catalyst, followed by neutralization with 12.3 parts by glacial acetic acid and reduction slowly with 705.5 parts of a deionised water. To this solubilized vehicle composition are then added 297 parts of a cationic pigment dispersion, 39 parts of ethylene glycol monohexyl ether, followed by 1886 parts of deionised water to let down the composition to a coating solids of about 20 percent by weight.

The crosslinker used is prepared by adding 218 parts by weight of 2-ethylhexanol slowly to 291 parts by weight of an 80/20 isomer mixture of 2,4-/2,6-toluene disocyanate, under agitation and under a dry nitrogen blanket, keeping the reaction temperature below 38^{o}C by external cooling. The mixture is held an additional 30 minutes at 38^{o}C and then heated to 60^{o}C, at which point 75 parts by weight of trimethylolpropane are added, followed by 0.08 part of dibutyl tin dilaurate catalyst. After an initial exotherm, the mixture is held at 121^{o}C for one and a half hours until all the isocyanate moiety is consumed, as indicated by infra-red scan. The mixture is then thinned with 249 parts of ethylene glycol monoethyl ether.

The cationic pigment dispersion is made by first preparing a dispersant by blending 138 parts of alkyl imidazoline, 31 parts of glacial acetic acid, 138 parts of ethylene glycol monobutyl ether and 383 parts of deionised water. 100 parts of this dispersion are mixed with 40 parts of acetylenic alcohol, 260 parts of deionised water, 134 parts of 325 mesh anthracite coal, 40 parts of lead silicate, 20 parts of strontium chromate and 6 parts of clay. The resultant slurry is then ground to Hegman No: 7 fineness in a suitable mill.

### Example 2 Production of modified Electrodip Lacquer

The procedure in Example 1 A) is repeated except that 12.5 parts by weight of (2-hydroxy-4-pentadecenylbenzyl)-2-mercapto benzothiazole, m.pt. 105-106^{o}C, disclosed on EP 259254, are included with the epoxide resin and the polycaprolactone diol. The final yield of the electrodip lacquer component so modified, is 637 parts by weight.

### Example 3 Production of an Electrodip Bath

The 70% resin obtained from Example 2 is firstly converted into a 32.7% pourable resin solution by adding propyleneglycolmonomethyl ether. The production of the electrodip bath is effected as follows:

To 430.3g of the 32.7% solution obtained as above, are added 77.5g of a blocked isocyanate, as, obtained in Example 1B), 3.3g dibutyl tin dilaurate and 10.2 g glacial acetic acid, in the given sequence. The charge is stirred for 30 minutes with a vaned stirrer, then 176.4 g deionised water are added, slowly with stirring, and stirring with the vaned stirrer is effected for a further 60 minutes. A further 350 g of deionised water are then added. The solids content of the electrodip bath so obtained is 18.3%.
Bath parameter: pH = 5.7, conductivity is 740 micro-siemens
Deposition parameter: bath temp. 27^{o}C, 200 volt/3 min.
Substrate: cold-rolled, untreated steel (Q-panel R46) cold-rolled, zinc-phosphated steel (RBo 28/NL 60/O).
Stoving conditions/layer thickness: the lacquer films are stoved at 200^{o}/25'. Films are produced having a layer thickness of 8-12 microns (Q- panel R46) or 7-11 microns (zinc-phosphated steel).
Weathering: the test sheets are subjected to a cyclic weathering test (GM Scab test). Weathering exposure time is 9 days (Q-panel R46) or 14 days (zinc-phosphated steel).
Results obtained are:

### 9 days GM - Scab Test

Control resin: (Example 1) moderate to severe surface rusting (12-13 mm delamination).
Resin of Example 2: no surface rust (20-22mm delamination)

### 14 days GM - Scab Test

Control resin: moderate surface rust (15-16 mm delamination)
Resin Example 2: no surface rust (7 mm delamination)
These results indicate that the method of the present invention leads to improved corrosion resistance, especially in the case of zinc-phosphated steel.

### Example 4 4-[4-hydroxy-α,α -dimethylbenzyl]-2-[(2-benzothiazolythio)methyl]phenol

22.8 Parts Bisphenol A, 33.4 parts 2-mercaptobenzothiazole, 6 parts paraformaldehyde, 2 parts di-n-butylamine and 250 parts toluene are heated at reflux for 3 hours. Evaporation of the resulting solution gives 45.4 parts 4-[4-hydroxy-α,α -dimethylbenzyl]-2-[(2-benzothiazolythio) methyl]phenol
'HNMR (in CDCl₃): δ1.6 ppm (6H); δ4.5 ppm (2H) δ6.6-8 ppm (11H);δ8.7 ppm (2H; replaceable)
Observed Elemental analysis: C, 64.93; H, 4.97; N, 4.13%

### Example 5

The procedure in Example 1(A) is repeated on 0.4 scale except that 12.5 parts by weight of the product of Example 4 are included with the epoxy resin and the polycaprolactone diol. The final yield of the electrodip lacquer component so modified is 787 parts by weight.

## Claims

1. A method for the manufacture of a corrosion-inhibiting surface coating comprising reacting a film-forming binder component of the surface coating medium with a compound having the formula IA or IB: 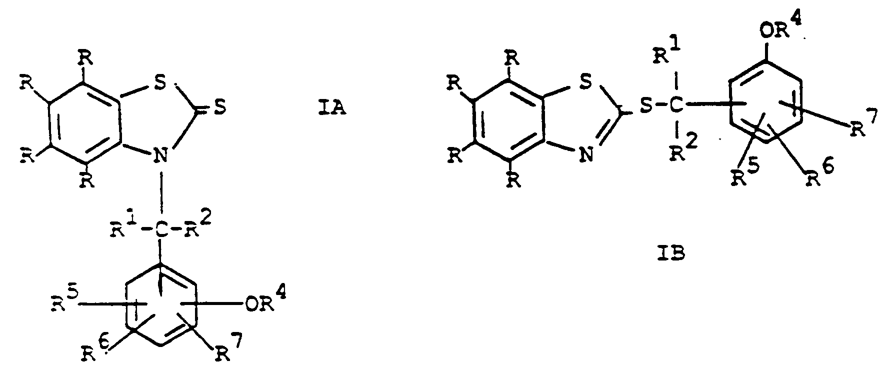 wherein each R, independently, is hydrogen, C₁-C₁₂ alkyl, C₃-C₂₀ alkenyl, C₁-C₄ halogenoalkyl, C₁-C₁₂ alkoxy, C₁-C₁₂ alkylthio, phenylthio, benzylthio, C₁-C₁₂ alkylsulphonyl, phenyl, C₇-C₁₅ alkylphenyl, C₇-C₁₀ phenylalkyl, C₅-C₈ cycloalkyl, halogen, nitro, cyano, carboxyl, COO(C₁-C₄ alkyl), hydroxy, amino, NHR³, N(R³)₂, CONH₂, CONHR³, CON(R³)₂, C₁-C₁₂ alkyl interrupted by an O-, N- or S-atom, NCO, C₁-C₄-alkylene-NCO, epoxy, C₁-C₄ alkylene epoxy, CHO or COR³;
R¹ is hydrogen, C₁-C₁₂ alkyl, phenyl optionally substituted by halogen, C₁-C₄ alkyl, C₁-C₄ alkoxy or nitro, or R¹ is pyridyl, thienyl or furyl;
R² is hydrogen or C₁-C₄ alkyl;
R³ is C₁-C₁₂ alkyl, C₃-C₁₂ alkyl interrupted by one or more O-atoms, C₅-C₈ cycloalkyl, benzyl, phenyl optionally substituted by halogen, C₁-C₄ alkyl, C₁-C₄ alkoxy or nitro, or
two groups R³, together with an N-atom to which they are attached, can form a pyrrolidino, piperidino or morpholino residue;
R⁴ is hydrogen, (CH₂)m CO₂H, (CH₂)mCO₂R⁸, 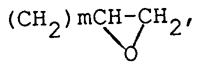 (CH₂)mNCO, (CH₂)mOH, (CH₂)mOSi(CH₃)₃, Si(CH₃)₃, SO₂R⁸, CONHR³, CON(R³)₂, C(=S)NHR³ or C(=S)N(R³)₂; m = integer from 1 to 20.
R⁵ and R⁶, independently, are hydrogen, halogen, C₁-C₄ alkoxy, cyano, nitro, C₁-C₂₀ alkyl, (CH₂)nCO₂R⁸, (CH₂)nCONHR³, (CH₂)nCON(R³)₂, C₃-C₂₀ alkenyl, C₇-C₁₀ phenylalkyl, phenyl, C₅-C₈ cycloalkyl or a group of formula IIA or IIB: 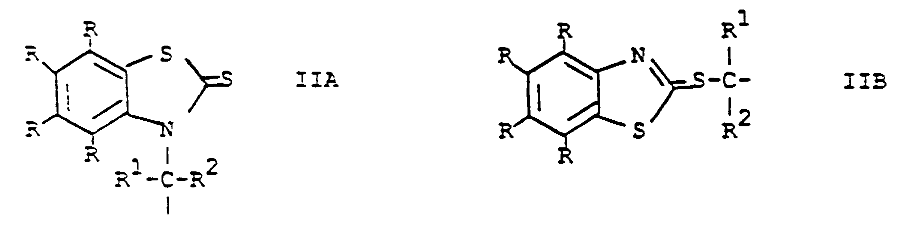 wherein R, R¹ and R² have their previous significance;
R⁷ is hydrogen, C₁-C₂₀ alkyl, C₁-C₂₀ alkyl interrupted by an O-, N- or S-atom; C₃-C₂₀ alkenyl, 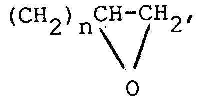 (CH₂)ₙ OH, hydroxy (CH₂)ₙNCO, (CH₂)ₙCO₂H, (CH₂)ₙCO₂R⁸, (CH₂)ₙSi(OCH₃)₃ or CHO or R⁷ is a residue of formula: 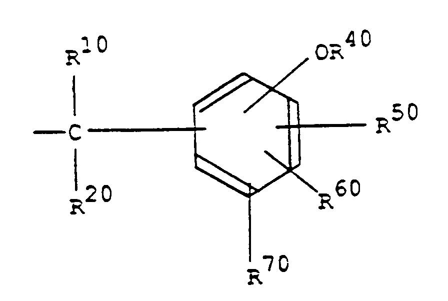 in which R¹⁰ has one of meanings of R¹ but does not necessarily have the identical meaning, R²⁰ has one of the meanings of R² but does not necessarily have the identical meaning, R⁴⁰ has one of the meanings of R⁴ but not necessarily the same meaning, R⁵⁰has one of the meanings, but not necessarily the same meaning as R⁵, R⁶⁰ has one of the meanings, but not necessarily the same meaning as R⁶, and R⁷⁰ has one of the meanings but not necessarily the same meaning as R⁷, or R⁵ and R⁷ , or R⁶ and R⁷ can form, fused to the phenol or phenoxy ring, a carbocyclic or heterocyclic ring, the heteroatoms being O, N or S, and the ring being optionally substituted with a C₁-C₄alkyl, C₁-C₄ alkoxy or halogen substituent, provided that, when R⁴ is hydrogen, R⁷ is one of the defined meanings other than hydrogen or C₁-C₂₀ alkyl;
R⁸ is C₁-C₂₀ alkyl optionally substituted by halogen or hydroxy, or is C₃-C₂₀ alkyl which is interrupted by one or more O-atoms and is substituted by one or more of hydroxy, phenyl, C₇-C₁₅ alkylphenyl or C₇-C₁₀ phenylalkyl; m is an integer from 1 to 20; and n is O or an integer from 1 to 20.

2. A method according to claim 1 wherein the film-forming binder component is an epoxide resin, a polyurethane resin, an aminoplast resin, or a mixture of such resins; or a basic aqueous dispersion or solution of an acidic resin.

3. A method according to claim 1 or 2 wherein the film-forming reactant is a binder for aqueous-based coating compositions.

4. A method according to claim 3 wherein the binder is an alkyd resin, an acrylic resin, a two-pack epoxy resin, a saturated polyester resin, a water-dilutable phenolic resin or dispersion thereof, a water-dilutable urea resin or a vinyl/acrylic resin.

5. A method according to claim 4 wherein the epoxide resin is one based on aromatic polyols.

6. A method according to claim 6 wherein the aromatic polyol is a bisphenol.

7. A method according to any of the previous claims wherein, in the compound of formula IA or IB, one R is hydrogen, C₁-C₄ alkyl, C₁-C₄ alkoxy, trifluoromethyl, halogen or nitro and the other three groups R are hydrogen.

8. A method according to any of the preceding claims wherein, in the compound of formula IA or IB, R¹ is hydrogen, C₁-C₈ alkyl, phenyl or furyl, and R² is hydrogen.

9. A method according to claim 8 in which R¹ and R² are hydrogen.

10. A method according to any of the preceding claims wherein, in the compound of formula IA or IB, R⁴ is as defined in claim 1, R⁵ and R⁶, independently, are hydrogen, C₁-C₈ alkyl, allyl, C₇-C₁₀ phenylalkyl, C₁-C₄ alkoxy, halogen, phenyl, cyclohexyl or a group -CH₂CH₂COOR⁸, in which R⁸ is C₁-C₁₈ alkyl or C₃-C₂₀ alkyl which is interrupted by one or more oxygen atoms, and R⁷ is hydrogen, C₁-C₁₈ alkyl or C₃-C₁₀ alkenyl, provided that when R⁴ is hydrogen, then R⁷ is C₃-C₁₈ alkenyl.

11. A method according to any of the preceding claims wherein one or more of a pigment, dye, extender and other conventional additive which is customary for coating compositions, is incorporated during the method of manufacture of the final surface coating medium.

12. A method according to claim 11 wherein a basic extender or pigment is incorporated.

13. A method according to claim 11 or 12 wherein a further organic, metal-organic or inorganic corrosion inhibitor is incorporated.

14. A method according to any of the preceding claims wherein the amount of a compound of formula IA or IB used ranges from 0.01-20 % by weight, based on the solids content of the coating medium.

15. A method according to any of the preceding claims wherein the surface coating medium so manufactured is an aqueous electrodepositable coating medium.

16. A surface coating medium when produced by a method claimed in any of the preceding claims.

17. Use of a surface coating medium according to claim 16 as a primer coating as a metal substrate.

18. Use according to claim 17 wherein the metal substrate is iron, steel, copper, aluminium, an aluminium alloy or zinc.

19. A compound having the formula IA or IB: 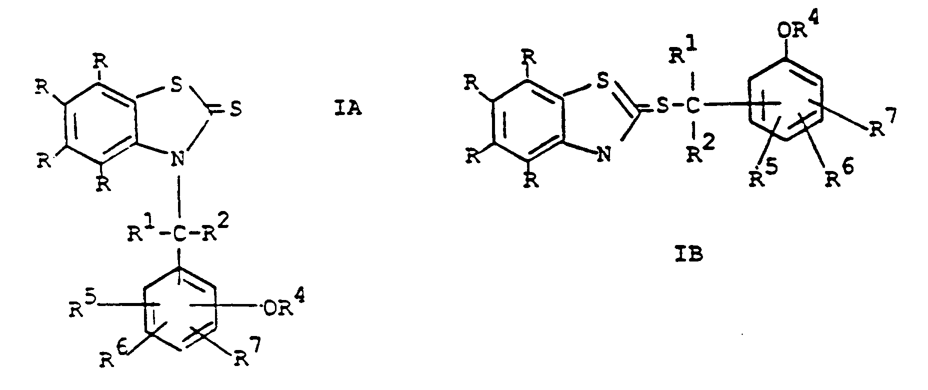 wherein each R, independently, is hydrogen, C₁-C₁₂ alkyl, C₃-C₂₀ alkenyl, C₁-C₄ halogenoalkyl, C₁-C₁₂ alkoxy, C₁-C₁₂ alkylthio, phenylthio, benzylthio, C₁-C₁₂ alkylsulphonyl, phenyl, C₇-C₁₅ alkylphenyl, C₇-C₁₀ phenylalkyl, C₅-C₈ cycloalkyl, halogen, nitro, cyano, carboxyl, COO(C₁-C₄ alkyl), hydroxy, amino, NHR³, N(R³)₂, CONH₂, CONHR³, CON(R³)₂, C₁-C₁₂ alkyl optionally interrupted by an O-, N- or S-atom, NCO, C₁-C₄ alkylene-NCO, epoxy, C₁-C₄ alkyleneepoxy, CHO or COR³;
R¹ is hydrogen, C₁-C₁₂ alkyl, phenyl optionally substituted by halogen, C₁-C₄ alkyl, C₁-C₄ alkoxy or nitro, or R¹ is pyridyl, thienyl or furyl;
R² is hydrogen or C₁-C₄ alkyl;
R³ is C₁-C₁₂ alkyl, C₃-C₁₂ alkyl interrupted by one or more O-atoms, C₅-C₈ cycloalkyl, benzyl, phenyl optionally substituted by halogen, C₁-C₄ alkyl, C₁-C₄ alkoxy or nitro, or two groups R³, together with an N-atom to which they are attached, can form a pyrrolidino, piperidino or morpholino residue;
R⁴ is hydrogen, (CH₂)m CO₂H, (CH₂)mCO₂R⁸, 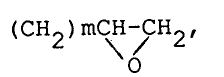 (CH₂)mNCO, (CH₂)mOH, (CH₂)mOSi(CH₃)₃, Si(CH₃)₃, SO₂R⁸, CONHR³, CON(R³)₂, C(=S)NHR³ or C(=S)N(R³)₂;
R⁵ and R⁶, independently, are hydrogen, halogen, C₁-C₄ alkoxy, cyano, nitro, C₁-C₂₀ alkyl, (CH₂)nCO₂R⁸, (CH₂)nCONHR³, (CH₂)nCON(R³)₂, C₃-C₂₀ alkenyl, C₇-C₁₀ phenylalkyl phenyl, C₅-C₈ cycloalkyl or a group of formula IIA or IIB: 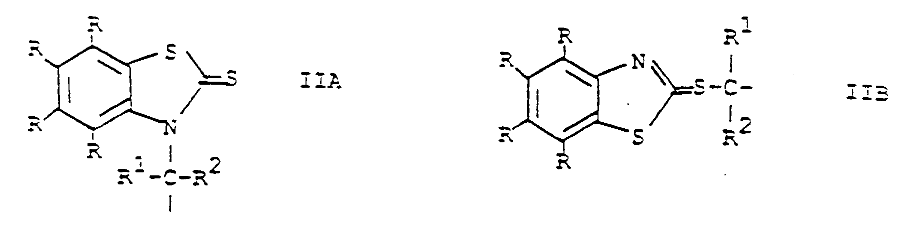 wherein R, R¹ and R² have their previous significance; R⁷ is hydrogen, C₁-C₂₀ alkyl, C₁-C₂₀ alkyl interrupted by an O-, N- or S-atom, C₃-C₂₀ alkenyl, 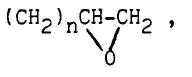 (CH₂)ₙOH, hydroxy (CH₂)ₙ NCO, (CH₂)ₙCO₂H, (CH₂)ₙCO₂R⁸, (CH₂)ₙSi(OH₃)₃ or CHO, or R⁷ is a residue of formula: 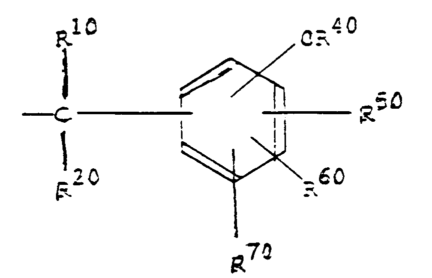 in which R¹⁰ has one of meanings of R¹ but does not necessarily have the identical meaning, R²⁰ has one of the meanings of R² but does not necessarily have the identical meaning, R⁴⁰ has one of the meanings of R⁴ but not necessarily the same meaning, R⁵⁰has one of the meanings, but not necessarily the same meaning as R⁵, R⁶⁰ has one of the meanings. but not necessarily the same meaning as R⁶, and R⁷⁰ has one of the meanings but not necessarily the same meaning as R⁷, or R⁵ and R⁷ , or R⁶ and R⁷ can form, fused to the phenol or phenoxy ring, a carbocyclic or heterocyclic ring, the heteroatoms being O, N or S, and the ring being optionally substituted with a C₁-C₄alkyl, C₁-C₄ alkoxy or halogen substituent, provided that, when R⁴ is hydrogen, R⁷ is one of the defined meanings other than hydrogen or C₁-C₂₀ alkyl;
R⁸ is C₁- C₂₀ alkyl optionally interrupted by halogen or hydroxy, or is C₃-C₂₀ alkyl which is interrupted by one or more O-atoms and is substituted by one or more O-atoms, and is substituted by one or more of hydroxy, phenyl, C₇-C₁₅ alkylphenyl or C₇-C₁₀ phenylalkyl; m is an integer from 1-20, and n is O or an integer from 1-20.
